# DEMANDE DE BREVET EUROPEEN

(11) **EP 1 082 956 A1**
(43) Date de publication de la demande: **14.03.2001**
(21) Numéro de dépôt: 00402218.2
(22) Date de dépôt: 03.08.2000
(51) Int. Cl.: A61K 7/48

(54) **Organogel comprenant un composé hydrophile sensible à l'oxydation et ses utilisations, notamment cosmétiques.**

(30) Priorité: 06.09.1999 FR 9911117
(71) Demandeur: L'OREAL, 75008 Paris (FR)
(72) Inventeur: Simonnet, Jean-Thierry, 75011 Paris (FR); Legret, Sylvie, 92320 Chatillon (FR)
(74) Mandataire: Renard, Emmanuelle

(57) **Abrégé**

La présente demande se rapporte à une composition sous forme d'émulsion comportant une phase huileuse dispersée, à l'aide d'un système tensioactif, dans une phase glycérolique renfermant un composé hydrophile sensible à l'oxydation et comprenant éventuellement au moins un glycol en plus de la glycérine, caractérisée en ce que le système tensioactif contient au moins un tensioactif susceptible de former une phase lamellaire au contact de la phase glycérolique et ayant un point de fusion supérieur ou égal à 35°C.

La composition obtenue a l'aspect d'un gel dans lequel le composé hydrophile est stabilisé vis-à-vis de l'oxydation, y compris dans des conditions aérobies.

L'invention se rapporte aussi à l'utilisation de ladite composition en vue de tonifier la peau et/ou de lisser les rides et ridules de la peau et/ou de lutter contre les méfaits des rayonnements UV et de la pollution et/ou de dépigmenter la peau.

## Description

La présente invention se rapporte à un organogel renfermant un composé hydrophile sensible à l'oxydation, c'est-à-dire à une composition sous forme d'émulsion comportant une phase huileuse dispersée, à l'aide d'un système tensioactif particulier, dans une phase glycérolique contenant ce composé hydrophile.

Il est connu d'introduire dans les compositions cosmétiques et/ou dermatologiques des actifs hydrophiles en vue d'apporter des traitements spécifiques à la peau.

Par exemple, l'acide ascorbique (ou vitamine C) est connu pour stimuler la synthèse du tissu conjonctif et notamment du collagène, renforcer les défenses du tissu cutané contre les agressions extérieures telles que les rayonnements ultraviolets et la pollution, compenser la déficience en vitamine E de la peau, dépigmenter la peau et posséder une fonction anti-radicaux libres. Ces deux dernières propriétés en font un excellent candidat comme actif cosmétique ou dermatologique pour lutter contre le vieillissement de la peau ou prévenir celui-ci.

En outre, il est connu également d'introduire dans les compositions cosmétiques des enzymes, et notamment des protéases et des lipases utilisées pour leurs propriétés protéolytiques et lipolytiques. Ces enzymes sont recherchées dans le domaine cosmétique pour leur pouvoir lissant et nettoyant, et leur aptitude à éliminer les cellules mortes de la peau.

Malheureusement, certains actifs, et en particulier ceux cités ci-dessus, sont instables du fait de leur sensibilité à des facteurs extérieurs tels que la lumière, la chaleur ou la présence d'oxygène soit de l'air, soit contenu dans l'eau. Ainsi, l'acide ascorbique se dégrade avec le temps dans les solutions aqueuses, y compris dans des conditions anaérobies, dans lesquelles il est susceptible de subir une succession de réactions d'hydrolyse et d'oxydation dépendant notamment du pH de la composition et de la présence d'ions métalliques servant de catalyseurs. Cette dégradation est bien plus rapide encore dans des conditions aérobies.

Cette instabilité va à l'encontre de l'efficacité recherchée et peut, de plus, être source de désagréments pour l'utilisateur, par exemple lorsque l'instabilité de l'actif entraîne des modifications de couleur et/ou d'odeur de la composition le contenant.

Aussi, différents moyens ont été envisagés pour stabiliser ces actifs. Par exemple, lorsque l'actif comporte un site réactif, notamment dans le cas de l'acide ascorbique, un des moyens pour le stabiliser consiste par exemple à bloquer ce site par estérification notamment avec des dérivés phosphatés et à utiliser ces dérivés à la place de l'actif libre. Malheureusement, ces dérivés présentent souvent une efficacité moins bonne que l'actif libre.

Il a été aussi envisagé d'utiliser des précurseurs de tels actifs, tels que des esters d'ose d'acide ascorbique qui, après application sur la peau, sont coupés par les enzymes cutanées et libèrent alors l'actif libre. Mais l'utilisation de tels dérivés ne permet pas toujours une libération rapide et en quantité suffisante d'actif à la surface de la peau.

Il reste donc souhaitable de pouvoir utiliser ces actifs sous leur forme d'actif libre, non modifié chimiquement. Or, compte tenu des problèmes d'instabilité mentionnés ci-dessus, la formulation de ces actifs impose des contraintes très lourdes. Il est ainsi nécessaire de formuler l'acide ascorbique à froid, sous atmosphère inerte et surtout de stocker les produits obtenus dans des conditionnements parfaitement étanches.

Il subsiste donc le besoin d'une composition dans laquelle des actifs cosmétiques hydrophiles sensibles à l'oxydation conserveraient toutes leurs propriétés et donc leur efficacité au cours du temps, y compris dans des conditions aérobies, de façon à réduire les contraintes de formulation et de conditionnement des produits contenant ces actifs et donc leur coût de revient .

La demanderesse a maintenant trouvé de manière inattendue qu'un support comprenant une phase huileuse dispersée, à l'aide d'un système tensioactif particulier, dans une phase glycérolique renfermant un composé hydrophile sensible à l'oxydation était susceptible de maintenir l'activité de cet actif sensible à l'oxygène de l'air et/ou à l'eau, et d'éviter ainsi sa dégradation.

Un tel support est encore appelé "organogel".

Le terme d'organogel a été initialement utilisé pour décrire un concept particulier de gélification par une solution de gélatine, d'une microémulsion inverse eau dans huile (voir Luisi et Al. Colloid & Polymer Science, 1990, vol. 268, p. 356-374). Le terme s'est étendu depuis peu à des systèmes gélifiés comportant deux phases non miscibles (eau dans huile) stabilisées par de la lécithine enrichie en Phosphatidyl Choline (appelée ci-après PC) et le plus souvent hydrogénée (voir Williman et al. Journal of Pharmaceutical Sciences, 1992, vol. 81, p. 871-874, et Schchipunov et al., Colloid Journal, 1995, vol. 57, p. 556-560). Ces émulsions présentent une phase lamellaire et sont sous forme de gels même en l'absence de gélifiants, d'où le nom d'organogels qui désignent ce type d'émulsion quel que soit le sens de l'émulsion (E/H ou H/E).

Ainsi, le document US-5,639,740 divulgue un organogel de lécithine sous la forme d'une émulsion huile-dans-eau, qui est destiné à favoriser la pénétration d'actifs tels que l'acide salicylique, les céramides et les α-hydroxy acides.

Par la suite, il a été développé des émulsions similaires, dans lesquelles l'eau était substituée par des polyols, donnant lieu à la formation d'émulsions huile dans glycol, stabilisées par de la lécithine hydrogénée. Pour diverses raisons et notamment des raisons de coût, on a ensuite essayé de remplacer dans les émulsions huile-dans-polyol, la lécithine hydrogénée enrichie en PC par d'autres composés.

Ainsi, le document US-5,587,149 décrit des émulsions comprenant une première phase à base de polyéthylène glycol, comprenant éventuellement en plus de la glycérine et/ou de l'eau et dans laquelle est dissoute de l'acide ascorbique, et une seconde phase à base d'huile de silicone, d'huiles de paraffine et/ou d'huiles végétales. L'émulsion comprend également un agent dispersant qui peut être un tensioactif siliconé ou un polysorbate 20 ou 80. Dans certaines conditions, le polysorbate 80 est susceptible de former des phases lamellaires. Toutefois, compte tenu du faible point de fusion de ce tensioactif (<0°C), ces phases lamellaires manquent de rigidité et il s'ensuit une certaine instabilité de l'émulsion. En outre, la stabilité de l'acide ascorbique n'est assurée que par l'encapsulation de cette émulsion dans une capsule de gélatine.

La demanderesse a maintenant découvert de façon surprenante que les organogels du type huile-dans-glycérol émulsifiés par des tensioactifs ayant un point de fusion supérieur ou égal à 35°C permettaient de stabiliser efficacement, y compris en milieu aérobie, l'acide ascorbique et les autres composés hydrophiles facilement oxydables. Ces organogels supportent ainsi l'introduction de quantités relativement imporantes d'eau (jusqu'à 20%) sans nuire à la stabilité des actifs qu'elles renferment, permettant ainsi leur formulation sous forme de fluides non gras. Ils conservent cependant une activité en eau suffisamment faible pour pouvoir être formulés sans conservateurs ce qui les rend bien adapté aux peaux sensibles.

La présente invention se rapporte ainsi à une composition sous forme d'émulsion comportant une phase huileuse dispersée, à l'aide d'un système tensioactif, dans une phase glycérolique renfermant un composé hydrophile sensible à l'oxydation, caractérisée en ce que le système tensioactif contient au moins un tensioactif susceptible de former une phase lamellaire au contact de la phase glycérolique et ayant un point de fusion supérieur ou égal à 35°C.

Les composés hydrophiles sensibles à l'oxydation par l'eau et/ou par l'oxygène de l'air qui peuvent être utilisés selon l'invention sont notamment l'acide ascorbique et ses dérivés hydrophiles, ainsi que les enzymes (par exemple lactoperoxydase, lipase, protéase, phospholipase, cellulase, catalase et superoxydismutase).

Les tensioactifs susceptibles de former une phase lamellaire au contact de la phase glycérolique, utilisés dans la composition de l'invention, sont amphiphiles et doivent ne pas être solubles dans l'eau. Ils ont de préférence un HLB (Hydrophilic Lipophilic Balance) allant de 1 à 10 et, mieux, de 2 à 8. On peut utiliser un tensioactif seul ou un mélange de tensioactifs. Quand on utilise un mélange de tensioactifs, les différents tensioactifs constituant le mélange peuvent avoir un HLB en dehors de la fourchette 1-10 du moment que le mélange de tensioactifs non ioniques a un HLB allant de 1 à 10.

Ces tensioactifs sont préférentiellement choisis parmi les esters gras de polyols, les éthers gras de polyols, les mélanges d'esters gras de polyols et les mélanges d'éthers gras de polyols. Les esters sont formés d'au moins un polyol et d'au moins un acide gras. L'acide gras peut comporter de 8 à 22 atomes de carbone et peut avoir une chaîne saturée ou non saturée, linéaire ou ramifiée. De préférence, l'acide gras a une chaîne saturée et linéaire. Cet acide gras est choisi de préférence parmi l'acide palmitique, l'acide stéarique et leurs mélanges. Les tensioactifs utilisés dans la composition de l'invention peuvent comporter de 1 à 10 chaînes et de préférence de 1 à 3 chaînes d'acides gras.

Les éthers gras de polyols sont formés d'au moins un polyol et d'au moins un alcool gras. L'alcool gras peut comporter de 8 à 22 atomes de carbone et peut avoir une chaîne saturée ou non saturée, linéaire ou ramifiée. De préférence, l'alcool gras a une chaîne saturée et linéaire. Cet alcool gras est choisi de préférence parmi l'alcool cétylique, l'alcool stéarylique, l'alcool laurylique et leurs mélanges.

Le polyol constitutif des tensioactifs utilisés dans la composition de l'invention est de préférence choisi dans le groupe formé par le glycérol, les polyglycérols, le sorbitan, les polysorbitans, les dérivés de sorbitan polyoxyéthylénés et notamment ceux comportant de 1 à 20 unités d'oxyde d'éthylène, les polyéthylènes (PEG) et notamment ceux comportant de 1 à 40 unités d'oxyde d'éthylène, le sucrose, le glucose, les dérivés oxyéthylénés du glucose et notamment ceux comportant de 1 à 20 unités d'oxyde d'éthylène, les polyglucoses, les polyglucoses oxyéthylénés et notamment ceux comportant de 1 à 20 unités d'oxyde d'éthylène, et leurs mélanges.

Les tensioactifs utilisés dans le cadre de la présente invention peuvent être notamment choisis parmi les dérivés polyoxyéthylénés des alcools cétylique, stéarylique et/ou laurylique, les esters de sorbitan et d'acide stéarique, les esters de sorbitan et d'acide palmitique, les esters de sucrose et d'acide stéarique, les esters de polyéthylène glycol de l'acide stéarique, les esters de glycérine ou de polyglycérine et de l'acide stéarique et leurs mélanges.

A titre d'exemple, on peut citer comme tensioactifs susceptibles de former une phase lamellaire avec la phase glycérolique, les composés suivants (en nom CTFA, International Cosmetic Ingredient Dictionary and Handbook) : Ceteth-2 (par exemple BRIJ 52 commercialisé par la société ICI), Steareth-2 (par exemple BRIJ 72 commercialisé par la société ICI), Sorbitan palmitate (par exemple SPAN 40 commercialisé par la société ICI), Sorbitan stearate (par exemple SPAN 60 commercialisé par la société ICI), Sorbitan tristearate (par exemple SPAN 65 commercialisé par la société ICI), PEG-8 stearate (par exemple MYRJ 45 commercialisé par la société chez ICI), Sucrose distearate qui est un mélange de mono, di et tri ester de sucrose (par exemple CRODESTA F10, CRODESTA F20, CRODESTA F50 commercialisés par la société Croda), Polyglyceryl-2 stearate (par exemple NIKKOL DGMS commercialisé par la société Nikko), Polyglyceryl-2 distearate (par exemple EMALEX PSGA commercialisé par la société Nikko), Polyglyceryl-3 distearate (par exemple EMALEX DGS 3 commercialisé par la société Nikko).

Un autre tensioactif non ionique utilisable dans la présente invention est la lécithine, de préférence hydrogénée, enrichie en phosphatidyl choline.

Selon l'invention, il est avantageux d'ajouter au(x) tensioactif(s) non ionique(s) au moins un additif choisi dans le groupe formé par :
- les stérols, et notamment les phytostérols et le cholestérol,
- les alcools et diols à longue chaîne,
- les amines à longue chaîne et leurs dérivés ammonium quaternaire.

Par rapport au(x) tensioactif(s) non ionique(s), la proportion de ces additifs n'excèdera pas, de préférence, 50% en poids.

En outre, le système tensioactif de la composition selon l'invention peut comprendre, au lieu des tensioactifs non ioniques mentionnés précédemment, un ou plusieurs tensioactifs ioniques. Un tensioactif ionique préféré dans ce cas est la lécithine enrichie en phosphatidyl triéthanolamine.

L'association de tensioactifs ioniques et non ioniques peut également être utile, dans certains cas, pour améliorer la stabilité de l'émulsion. Outre la lécithine enrichie en phosphatidyl triéthanolamine, les tensioactifs ioniques utilisables à cette fin peuvent être choisis parmi les tensioactifs anioniques de préférence neutralisés, les dérivés alkylsulfoniques, les tensioactifs cationiques et leurs mélanges. Toutefois, le tensioactif ionique ne doit pas modifier la capacité du tensioactif non ionique à former une phase lamellaire. Les tensioactifs anioniques sont plus particulièrement choisis dans le groupe formé par :
- le dicetylphosphate, le dimyristylphosphate et leurs sels alcalins ;
- les sels alcalins du cholestérol sulfate ;
- les sels alcalins du cholestérol phosphate ;
- les lipoaminoacides tels que les acylglutamates mono- et di-sodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les phospholipides.

Les dérivés alkylsulfoniques peuvent être plus particulièrement choisis parmi les dérivés alkylsulfoniques de formule (I) : 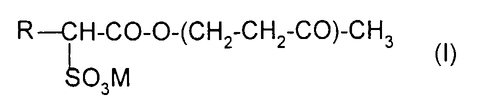 dans laquelle R représente un radical alkyle comportant de 16 à 22 atomes de carbone, en particulier les radicaux C₁₆H₃₃ et C₁₈H₃₇ pris en mélange ou séparément, et M est un métal alcalin tel que le sodium.

Les tensioactifs cationiques peuvent être choisis, par exemple dans le groupe formé par les sels d'ammonium quaternaire, les amines grasses et leurs sels.

Les sels d'ammonium quaternaires sont par exemple :
- ceux qui présentent la formule générale (II) suivante : 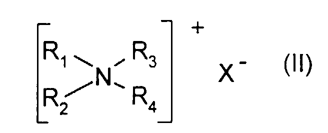 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl-ou-alkylarylsulfonates. Comme sels d'ammonium quaternaire de formule (II), on préfère, d'une part, les chlorures de tétraalkylammonium comme par exemple les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthyl-ammonium, de cétyltriméthyl-ammonium, de benzyl diméthyl stéaryl-ammonium ou encore, d'autre part, le chlorure de stéaramidopropyldiméthyl (myristyl acetate) ammonium vendu sous la dénomination CERAPHYL 70 par la société Van Dyk.
- les sels d'ammonium quaternaire de l'imidazolinium, comme par exemple ceux de formule (III) suivante : 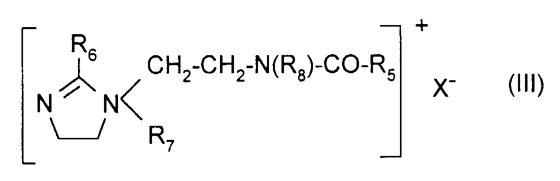 dans laquelle R₅ représente un radical alcényle ou alkyle comportant de 8 à 30 atomes de carbone, par exemple dérivé des acides gras du suif ; R₆ représente un atome d'hydrogène, un radical alkyle comportant de 1 à 4 atomes de carbone ou un radical alcényle ou alkyle comportant de 8 à 30 atomes de carbone ; R₇ représente un radical alkyle comportant de 1 à 4 atomes de carbone ; R₈ représente un atome d'hydrogène, un radical alkyle comportant de 1 à 4 atomes de carbone ; X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone, par exemple dérivés des acides gras du suif, R₇ désigne un radical méthyle, R₈ désigne l'hydrogène. Un tel produit est par exemple vendu sous la dénomination REWOQUAT W 75 par la société Rewo.
- les sels de diammonium quaternaire de formule (IV) : 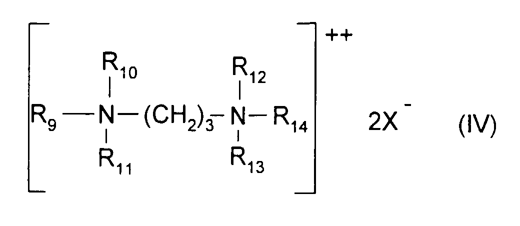 dans laquelle R₆ désigne un radical aliphatique comportant environ de 16 à 30 atomes de carbone ; R₇, R₈, R₉, R₁₀, et R₁₁ sont choisis parmi l'hydrogène ou un radical alkyle comportant de 1 à 4 atomes de carbone ; et X est un anion choisi dans le groupe des halogénures, acétates, phosphates, nitrates et méthylsulfates. De tels sels de diammonium quaternaire comprennent notamment le dichlorure de propanesuif diammonium.

Le ou les tensioactifs ioniques, quand ils sont présents, peuvent l'être en une quantité d'au maximum 20 % en poids par rapport au poids de tensioactif(s) non ionique(s) utilisé(s). Quand la composition en contient, la quantité de tensioactif(s) ionique(s) va de préférence de 0,01 à 5 % en poids par rapport au poids total du système tensioactif.

En outre, dans la composition de l'invention, la quantité du système tensioactif va de préférence de 0,1 à 4,25 % et mieux de 0,5 à 3 % en poids par rapport au poids total de la composition.

Le rapport en poids phase glycérolique/système tensioactif est avantageusement égal ou supérieur à 20 et va de préférence de 20 à 50.

La phase glycérolique contient au moins de la glycérine. Dans une forme de réalisation préférée de l'invention, elle contient en outre au moins un glycol. Une telle association permet d'améliorer la stabilité de l'émulsion à long terme.

Les glycols utilisés dans la composition de l'invention peuvent être choisis par exemple parmi le propylène glycol, le 1,3-butylène glycol, le dipropylène glycol, le pentylène glycol, l'isoprène glycol et les polyéthylène glycols notamment ceux comportant 4 à 16 unités d'oxyde d'éthylène et de préférence de 8 à 12 unités d'oxyde d'éthylène, et leurs mélanges.

La phase glycérolique représente, dans la composition de l'invention, au moins 30 % en poids par rapport au poids total de la composition, et de préférence de 30 à 85 % et mieux de 40 à 75 % en poids par rapport au poids total de la composition. Selon un mode préféré de réalisation de l'invention, la glycérine et le ou les glycols éventuels sont présents en un rapport en poids glycérine/glycols allant de 0,5 à 5 et de préférence de 1 à 5.

Il est possible d'introduire de l'eau dans l'émulsion de l'invention. De préférence, la quantité d'eau est telle que l'activité en eau (aw) de la composition finale n'est pas supérieure à 0,7. On peut utiliser différentes méthodes pour mesurer l'activité en eau d'une composition, la plus courante étant la méthode manométrique par laquelle on mesure directement la pression de vapeur. De préférence, la quantité d'eau dans la composition de l'invention est au maximum de 20 % en poids par rapport au poids total de la composition.

La phase huileuse de la composition selon l'invention représente généralement de 5 à 45 % et de préférence de 15 à 35 % en poids par rapport au poids total de la composition.

La nature de la phase huileuse de l'émulsion selon l'invention n'est pas critique. La phase huileuse peut ainsi être constituée par tous les corps gras et notamment les huiles, classiquement utilisés dans les domaines cosmétique ou dermatologique.

Parmi les huiles utilisables dans l'émulsion de l'invention, on peut citer par exemple les huiles d'origine végétale, telles que les huiles de jojoba, avocat, amande douce, abricot, maïs et la fraction liquide de beurre de karité ; les huiles minérales comme l'huile de vaseline ; les huiles de synthèse comme les triglycérides (par exemple caprylic/capric triglycerides), le palmitate d'éthyl-2 hexyle, le myristate d'isopropyle, l'isoparaffine hydrogénée, l'isononanoate d'isononyle, l'octanoate de cétéaryle ; les huiles de silicone volatiles ou non volatiles et les huiles fluorées. Les autres corps gras susceptibles d'être présents dans la phase huileuse peuvent être par exemple les acides gras, les alcools gras, les cires et les lipides tels que les céramides.

La composition selon l'invention peut constituer notamment une composition cosmétique ou dermatologique et contient alors un milieu physiologiquement acceptable, c'est-à-dire compatible avec la peau, les lèvres, le cuir chevelu, les yeux et/ou les cheveux.

De façon connue, les compositions de l'invention peuvent contenir des adjuvants habituels dans les domaines considérés, tels que des actifs hydrophiles et/ou lipophiles (par exemple le tocophérol et ses dérivés tels que l'acétate de tocophéryle ; le rétinol et ses esters tels que le palmitate de rétinyle ; les flavonoïdes ou extraits de plantes en contenant ; etc.), des conservateurs, des antioxydants, des parfums, des solvants, des charges, des filtres, des matières colorantes, des agents basiques ou acides et encore des vésicules lipidiques. Ces adjuvants sont utilisés dans les proportions habituelles dans les domaines considérés, et par exemple de 0,01 à 30 % du poids total de la composition, et ils sont, selon leur nature, introduits dans l'une ou l'autre phase de l'émulsion, ou encore dans des vésicules. Ces adjuvants ainsi que leurs concentrations doivent être tels qu'ils ne modifient pas la propriété recherchée pour l'émulsion de l'invention.

L'addition de charges permet de modifier, si besoin est, la texture de la composition. Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; et leurs mélanges. Ces charges peuvent être présentes dans une quantité allant de 0 à 10 % en poids et de préférence de 0,5 à 4 % en poids par rapport au poids total de la composition.

Le système tensioactif utilisé dans la composition de l'invention permet d'obtenir une émulsion stable. Toutefois, selon la quantité et la nature du tensioactif, on peut être amené à corriger les variations de viscosité par l'introduction de gélifiants. Ainsi, il est possible d'introduire dans la composition de l'invention, un gélifiant hydrophile ou lipophile, à la condition qu'il soit en mesure de gonfler dans la phase dans laquelle il est dispersé. Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles, l'ADN et les argiles, et comme gélifiants lipophiles, on peut citer les argiles modifiées comme les bentones, les sels métalliques d'acides gras, la silice hydrophobe et les polyéthylènes.

Les compositions selon l'invention sont opalescentes à opaques et peuvent être plus ou moins fluides. Elles peuvent donc se présenter sous forme de sérum, de lait, de crème ou de pâte. Ces compositions sont préparées selon les méthodes usuelles.

Les compositions objet de l'invention trouvent notamment leur application dans un grand nombre de traitements cosmétiques de la peau, des lèvres et des cheveux, y compris le cuir chevelu, notamment pour le traitement, la protection, le soin, le démaquillage et/ou le nettoyage de la peau, des lèvres et/ou des cheveux, et/ou pour le maquillage de la peau et/ou des lèvres.

Aussi, l'invention a encore pour objet l'utilisation cosmétique de la composition telle que définie ci-dessus pour tonifier la peau et/ou lisser les rides et ridules de la peau et/ou lutter contre les méfaits des rayonnements UV et de la pollution et/ou dépigmenter la peau.

L'invention a également pour objet un procédé de traitement cosmétique de la peau, caractérisé en ce qu'on applique sur la peau une composition telle que définie ci-dessus.

L'invention a également pour objet l'utilisation de la composition telle que définie ci-dessus pour la fabrication d'une préparation destinée à tonifier la peau et/ou lisser les rides et ridules de la peau et/ou lutter contre les méfaits des rayonnements UV et de la pollution et/ou dépigmenter la peau.

L'invention a encore pour objet un procédé de stabilisation d'un composé hydrophile sensible à l'oxydation, comprenant les étapes consistant :
(a) à introduire dans du glycérol éventuellement additionné de glycol(s) et/ou d'eau un tensioactif susceptible de former une phase lamellaire au contact du glycérol et ayant un point de fusion d'au moins 35°C,
(b) à ajouter ledit composé hydrophile au mélange précédent,
(c) à incorporer au moins une huile au mélange ainsi obtenu, pour former une émulsion, et
(d) à soumettre l'émulsion résultante à une homogénéisation grande vitesse, à une température supérieure d'environ 5°C à la température de transition de phase dudit tensioactif.

Les exemples qui suivent permettront de mieux comprendre l'invention, sans toutefois présenter un caractère limitatif. Les quantités indiquées sont en pourcentage pondéral, sauf mention contraire.

### Exemple 1 : Organogel

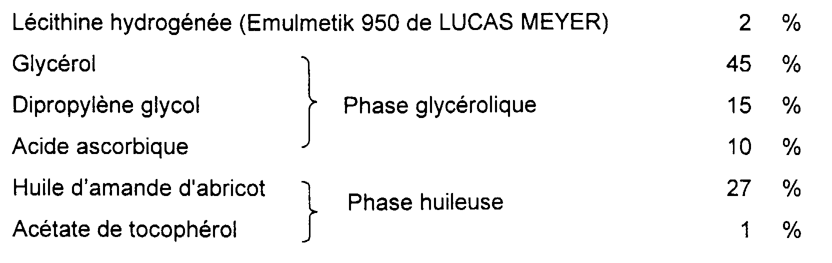

### Mode opératoire :

La lécithine hydrogénée est introduite sous vive agitation à 75°C dans le mélange de glycérol et de dipropylène glycol. L'agitation est maintenue dans les mêmes conditions pendant 30 minutes. L'acide ascorbique est ensuite introduit entre 70 et 65°C et l'agitation est maintenue jusqu'à parfaite solubilisation de celui-ci. La phase huileuse est ensuite ajoutée, toujours dans les mêmes conditions qui sont maintenues pendant encore une heure. Puis, on arrête le chauffage et on réduit la vitesse d'agitation jusqu'à ce que la température soit descendue à 60°C. On passe ensuite le mélange à l'homogénéisateur rotor-stator grande vitesse (Ultraturax) à environ 20 000 tours/minute pendant 2 minutes à une température supérieure d'environ 5°C à la température de transition de phase du tensioactif dans la phase glycérolique, comprise pour la lécithine hydrogénée entre 50 et 55°C. Le réseau lamellaire se forme dans les 24 heures.

On obtient un gel fluide, très fin. Sa valeur d'activité en eau a_{w} est de 0,1445. Le fluide est stable deux mois à 4°C, 25°C, 37°C et 45°C, c'est-à-dire qu'on n'observe pas de crémage, de sédimentation ou de séparation de phase de l'émulsion. En outre, un dosage par HPLC de l'acide ascorbique montre une perte de seulement 5% d'acide ascorbique après deux mois de stockage à 45°C, alors qu'aucune précaution particulière n'a été prise lors de la préparation du fluide ou de sa conservation.

Ce fluide peut notamment être utilisé pour le soin de la peau, en vue de prévenir et/ou de lutter contre les signes du vieillissement.

### Exemple 2 : Organogel

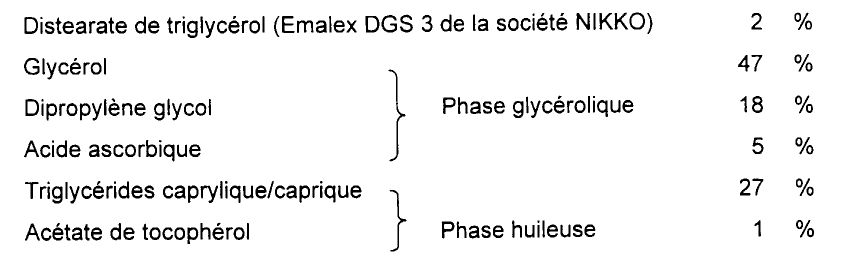

Cette composition est préparée de la même manière que dans l'Exemple 1, excepté que la température de passage à l'homogénéisateur grande vitesse est comprise entre 45 et 55°C.

On obtient un gel fluide, très fin. Sa valeur d'activité en eau a_{w} est de 0,1. Le fluide est stable deux mois à 4°C, 25°C, 37°C et 45°C, c'est-à-dire qu'on n'observe pas de crémage, de sédimentation ou de séparation de phase de l'émulsion. En outre, un dosage par HPLC de l'acide ascorbique montre une perte de seulement 3,6% d'acide ascorbique après deux mois de stockage à 45°C, alors qu'aucune précaution particulière n'a été prise lors de la préparation du fluide ou de sa conservation.

Ce fluide peut notamment être utilisé pour le soin de la peau, en vue de prévenir et/ou de lutter contre les les agressions de l'environnement, en particulier des UV et de la pollution.

### Exemple 3 : Organogel

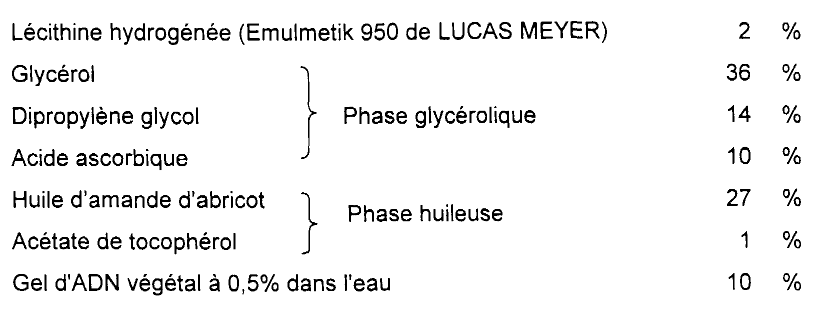

Le mode opératoire est identique à celui de l'Exemple 1, excepté que le gel d'ADN est ajouté à 55°C sous agitation, après l'homogénéisation au rotor-stator grande vitesse. Une fois la dispersion du gel réalisée, l'agitation est interrompue jusqu'au refroidissement à température ambiante du mélange.

On obtient un organogel légèrement coulant, opalescent et fin, parfaitement stable deux mois à 4°C, 25°C, 37°C et 45°C, c'est-à-dire qu'on n'observe pas de crémage, de sédimentation ou de séparation de phase de l'émulsion. En outre, un dosage par HPLC de l'acide ascorbique montre une perte de seulement 10% d'acide ascorbique après deux mois de stockage à 45°C, alors qu'aucune précaution particulière n'a été prise lors de la préparation du fluide ou de sa conservation.

## Revendications

1. Composition sous forme d'émulsion comportant une phase huileuse dispersée, à l'aide d'un système tensioactif, dans une phase glycérolique renfermant un composé hydrophile sensible à l'oxydation, caractérisée en ce que le système tensioactif contient au moins un tensioactif susceptible de former une phase lamellaire au contact de la phase glycérolique et ayant un point de fusion supérieur ou égal à 35°C.

2. Composition selon la revendication 1, caractérisée en ce que le composé hydrophile est choisi parmi l'acide ascorbique et ses dérivés hydrophiles, et les enzymes.

3. Composition selon la revendication 1 ou 2, caractérisée en ce que le tensioactif est un tensioactif non ionique choisi parmi la lécithine hydrogénée enrichie en phosphatidyl choline, les esters gras de polyols, les éthers gras de polyols, les mélanges d'esters gras de polyols et les mélanges d'éthers gras de polyols.

4. Composition selon la revendication 3, caractérisée en ce que l'ester gras de polyol est formé d'au moins un polyol et d'au moins un acide gras comportant de 8 à 22 atomes de carbone et ayant une chaîne saturée ou non saturée, linéaire ou ramifiée.

5. Composition selon la revendication 4, caractérisée en ce que l'acide gras est choisi parmi l'acide palmitique, de l'acide stéarique et leurs mélanges.

6. Composition selon la revendication 3, caractérisée en ce que l'éther gras de polyol est formé d'au moins un polyol et d'au moins un alcool gras comportant de 8 à 22 atomes de carbone et ayant une chaîne saturée ou non saturée, linéaire ou ramifiée.

7. Composition selon la revendication 6, caractérisée en ce que l'alcool gras est choisi parmi l'alcool cétylique, l'alcool stéarylique, l'alcool laurylique et leurs mélanges.

8. Composition selon l'une quelconque des revendications 3 à 7, caractérisée en ce que l'ester gras de polyol ou l'éther gras de polyol sont formés à partir d'un polyol choisi dans le groupe comprenant le glycérol, les polyglycérols, le sorbitan, les polysorbitans, les dérivés de sorbitan polyoxyéthylénés, les polyéthylènes, le sucrose, le glucose, les dérivés oxyéthylénés du glucose, les polyglucoses, les polyglucoses oxyéthylénés et leurs mélanges.

9. Composition selon l'une quelconque des revendications1 à 8, caractérisée en ce que le tensioactif non ionique est choisi parmi les dérivés polyoxyéthylénés des alcools cétylique, stéarylique et/ou laurylique, les esters de sorbitan et d'acide stéarique, les esters de sorbitan et d'acide palmitique, les esters de sucrose et d'acide stéarique, les esters de polyéthylène glycol de l'acide stéarique, les esters de glycérine ou de polyglycérine et d'acide stéarique, et leurs mélanges.

10. Composition selon l'une quelconque des revendications 1 à 9, caractérisée en ce qu'elle comprend en outre au moins un additif choisi dans le groupe formé par :
- les stérols, et notamment les phytostérols et le cholestérol,
- les alcools et diols à longue chaîne, et
- les amines à longue chaîne et leurs dérivés ammonium quaternaire.

11. Composition selon la revendication 10, caractérisée en ce que la quantité de tensioactif(s) ionique(s) est d'au maximum 20 % en poids par rapport au poids de tensioactif non ionique susceptible de former une phase lamellaire.

12. Composition selon la revendication 1 ou 2, caractérisée en ce que le tensioactif est un tensioactif ionique, tel que la lécithine enrichie en phosphatidyl triéthanolamine.

13. Composition selon l'une quelconque des revendications 1 à 12, caractérisée en ce que la quantité du système tensioactif va de 0,1 à 4,25 % en poids par rapport au poids total de la composition.

14. Composition selon l'une quelconque des revendications 1 à 13, caractérisée en ce que la phase glycérolique renferme en outre au moins un glycol.

15. Composition selon la revendication 14, caractérisée en ce que le glycol est choisi parmi le propylène glycol, le 1,3-butylène glycol, le dipropylène glycol, le pentylène glycol, l'isoprène glycol et les polyéthylène glycols et leurs mélanges.

16. Composition selon la revendication 14 ou 15, caractérisée en ce que le rapport en poids glycérine/glycol(s) va de 0,5 à 5.

17. Composition selon l'une quelconque des revendications 1 à 16, caractérisée en ce que la phase glycérolique représente de 30 à 85 % en poids par rapport au poids total de la composition.

18. Composition selon l'une quelconque des revendications 1 à 17, caractérisée en ce qu'elle contient de l'eau.

19. Composition selon l'une quelconque des revendications 1 à 18, caractérisée en ce qu'elle comprend en outre un gélifiant.

20. Composition selon l'une quelconque des revendications 1 à 19, caractérisée en ce que la phase huileuse représente de 5 à 45 % en poids par rapport au poids total de la composition.

21. Utilisation cosmétique de la composition selon l'une quelconque des revendications 1 à 20, en vue de tonifier la peau et/ou de lisser les rides et ridules de la peau et/ou de lutter contre les méfaits des rayonnements UV et de la pollution et/ou de dépigmenter la peau.

22. Procédé de traitement cosmétique de la peau, caractérisé en ce qu'on applique sur la peau une composition selon l'une quelconque des revendications 1 à 20.

23. Utilisation de la composition selon l'une quelconque des revendications 1 à 20, pour la fabrication d'une préparation destinée à tonifier la peau et/ou lisser les rides et ridules de la peau et/ou lutter contre les méfaits des rayonnements UV et de la pollution et/ou dépigmenter la peau.

24. Procédé de stabilisation d'un composé hydrophile sensible à l'oxydation, comprenant les étapes consistant :
(a) à introduire dans du glycérol éventuellement additionné de glycol(s) et/ou d'eau un tensioactif susceptible de former une phase lamellaire au contact du glycérol et ayant un point de fusion d'au moins 35°C,
(b) à ajouter ledit composé hydrophile au mélange précédent,
(c) à incorporer au moins une huile au mélange ainsi obtenu, pour former une émulsion, et
(d) à soumettre l'émulsion résultante à une homogénéisation grande vitesse, à une température supérieure d'environ 5°C à la température de transition de phase dudit tensioactif.
